# EUROPÄISCHE PATENTSCHRIFT

(11) **EP 0 540 509 B1**
(45) Veröffentlichungstag und Bekanntmachung des Hinweises auf die Patenterteilung: **21.09.1994**
(21) Anmeldenummer: 89911243.7
(22) Anmeldetag: 17.10.1989
(51) Int. Cl.: B61L 27/04, B61L 23/00

(54) **ÜBERWACHUNGSANLAGE ZUM NACHRÜSTEN EINER FÖRDERANLAGE**
A SUPPLEMENTARY MONITORING INSTALLATION FOR A CONVEYOR
DISPOSITIF DE CONTROLE POUR L'EQUIPEMENT COMPLEMENTAIRE D'UNE INSTALLATION DE TRANSPORT

(30) Priorität: 20.10.1988 CH 3910/88
(43) Veröffentlichungstag der Anmeldung: 12.05.1993
(73) Patentinhaber: Büro Patent AG, CH-8750 Glarus (CH)
(72) Erfinder: ZIMMERMANN, Rolf, D-8000 München 70 (DE)
(74) Vertreter: Schmauder, Klaus Dieter
(86) Internationale Anmeldenummer: CH8900182
(87) Internationale Veröffentlichungsnummer: WO9004532

(56) Entgegenhaltungen:
- EP-A- 0 127 566
- EP-A- 0 164 302
- US-A- 3 502 038
- US-A- 4 056 063

## Beschreibung

Die Erfindung betrifft eine Förderanlage gemäss dem Oberbegriff des Anspruches 1.

Förderanlagen der im Oberbegriff des Anspruches 1 genannten Art sind mehrfach bekannt, so beispielsweise aus den US-A-3 340 821 und US-A-3 502 038. Bei einer solchen Förderanlage bestehen die Zielcodes aus einzelnen oder mehreren Elektromagneten, die am Förderwagen angeordnet sind und in Form des Zielcodes eingestellt werden. Am beweglichen Führungsschienenteil von Weichen sind Empfangseinrichtungen angeordnet, welche die Zielcodes der Förderwagen während deren Fahrt abtasten und die Weichensteuereinrichtung in Abhängigkeit vom jeweils abgetasteten Zielcode unmittelbar beeinflussen. Solche Förderanlagen funktionieren gut, jedoch ist eine Ueberwachung von deren Betriebszustand, beispielsweise ob ein abgesandter Förderwagen auch tatsächlich die eingestellte Zielstation richtig erreicht, nicht möglich.

Andererseits sind moderne Förderanlagen beispielsweise aus der EP-OS 0 164 302 bekannt, die eine zentrale Steuereinheit enthalten, welche nicht nur der Steuerung der Förderwagen an die eingestellte Zielstation ermöglichen, sondern gleichzeitig auch eine Ueberwachung der Förderanlage gewährleisten, wobei Förderwagen mit individuellen Identifikationscodes versehen sind, die ebenfalls abgetastet und der zentralen Steuereinheit zugeführt werden. In der zentralen Steuereinheit werden die Identifikationscodes und die Zielcodes verarbeitet und dienen zur Steuerung der Weichen und Ueberwachung der Förderanlage. Eine solche Steuereinrichtung ist zum Nachrüsten einer bestehenden Förderanlage der oben genannten Art nicht geeignet.

Aufgabe der Erfindung ist es, eine Förderanlage gemäß dem Oberbegriff des Anspruchs 1 zu schaffen, in der die bisherige Steuerung eines Förderwagens mittels des Zielcodes an seine Zielstation beibehalten wird und lediglich zusätzlich eine Ueberwachung der Förderanlage möglich ist.

Die gestellte Aufgabe wird durch die Merkmale des Anspruches 1 gelöst. Damit ist es möglich, bestehende Förderanlagen nachzurüsten und ihnen die Eigenschaften moderner Förderanlagen zu verleihen, ohne dass das bisherige Steuersystem geändert wird. Das bisherige Steuersystem der Förderanlage und das ergänzte Ueberwachungssystem können grundsätzlich unabhängig voneinander bestehen, sich jedoch gegebenenfalls zusätzlich zu ergänzen. Mit der nachgerüsteten Ueberwachungsanlage kann der Weg des Förderwagens von der Sendestation bis zur Empfangsstation überwacht und mittels Codestellvorrichtung beispielsweise ein Förderwagen auf dem kürzesten Weg umprogrammiert oder ein in der Förderanlage irrender Förderwagen beispielsweise an die Sendestation zurückgeführt oder an eine zentrale Sammelstation weitergeleitet werden.

Bevorzugte Ausführungsformen der Förderanlage sind in den Ansprüchen 2 bis 7 beschrieben.

Grundsätzlich kann es genügen, bei einfachen Förderanlagen lediglich die Stationen mit Empfangsgeräten der genannten Art auszurüsten. Bei komplizierteren Förderanlagen empfiehlt sich eine Ausgestaltung nach Anspruch 2, da dann die Förderwagen auch während ihrer Fahrt längs der Förderspur laufend überwacht werden können. Solche längs der Förderspur angeordneten Empfänger können gegebenenfalls nur Leseeinrichtungen für Identifikationscodes aufweisen, enthalten vorzugsweise aber auch Leseeinrichtungen für Zielcodes.

Zweckmässig ist es auch, wenn die Empfangsgeräte Mikroprozessoren enthalten, welche die empfangenen Signale verarbeiten und so dem Zentralrechner zuführen. Dadurch wird insbesondere auch eine Ausgestaltung nach Anspruch 4 möglich, sodass die Ueberwachungsanlage bei Fehlfunktionen aktiv in das Geschehen der Förderanlage eingreifen kann. Dabei ist eine Ausgestaltung nach Anspruch 5 zweckmässig, sodass beispielsweise bei einer Besetztmeldung der Zielstation oder bei falsch eingestelltem Zielcode der Absendevorgang des Förderwagens an der Sendestation unterbrochen werden kann. Besonders vorteilhaft ist eine Ausgestaltung nach Anspruch 6.

Besonders vorteilhaft ist auch eine Ausgestaltung nach Anspruch 7, die es ermöglicht, den Funktionsverlauf der Förderanlage an einem Bildschirm darzustellen oder Fehlfunktionen anzuzeigen oder ein Betriebsprotokoll der Förderanlage zu erstellen.

Ausführungsbeispiele der Erfindung werden nachfolgend anhand schematischer Zeichnungen näher beschrieben, dabei zeigen:
- Figur 1: einen Ausschnitt aus einer Förderanlage, die mit einer Ueberwachungsanlage ausgestattet ist, und

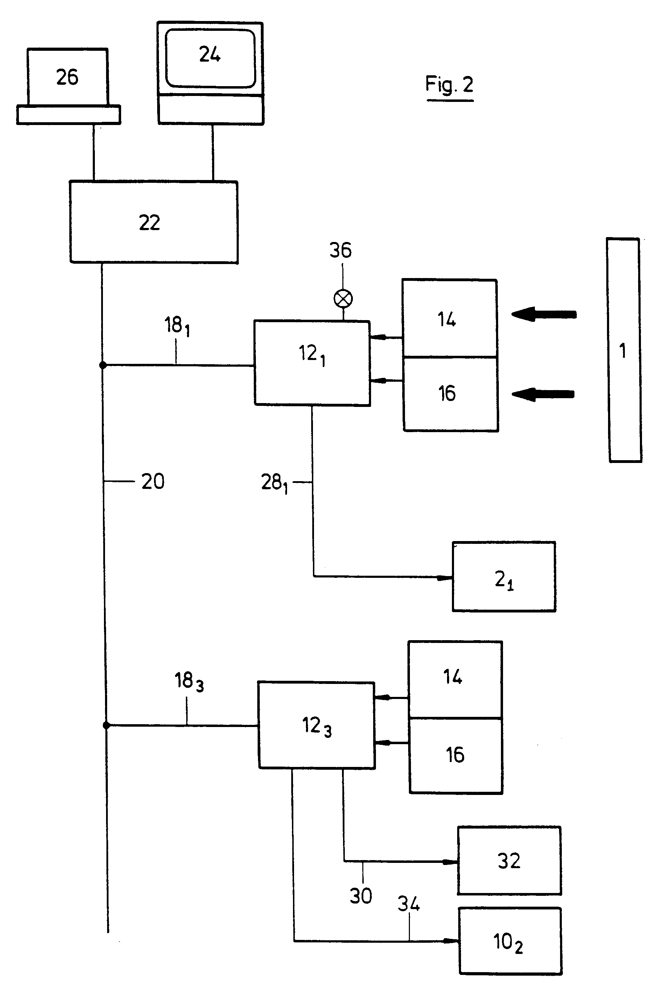

- Figur 2: das Blockschaltbild der Ueberwachungsanlage der Figur 1.

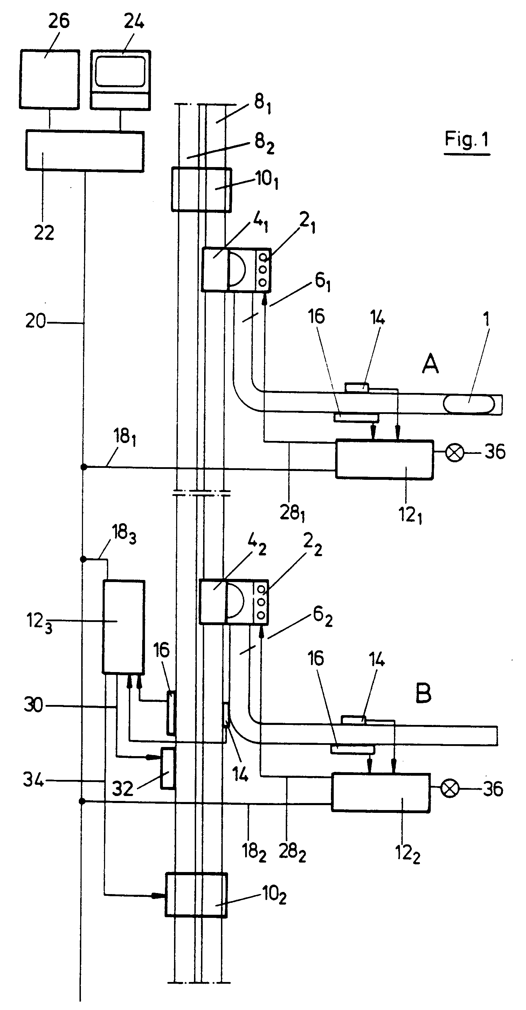

Die in Figur 1 im Ausschnitt dargestellte Förderanlage entspricht jener der US-PSen 3 340 821 und 3 502 038, die in ihrem Variationsreichtum beispielsweise gemäss Figur 10 der EP-OS 0 164 302 ausgestaltet sein kann.

Bei einer solchen Förderanlage werden die Zielcodes an den Stationen A und B durch Schiebemagnete am Förderwagen 1 eingestellt und durch entsprechende Abtasteinrichtungen 2₁,2₂ an den Weichen 4₁,4₂ abgetastet, worauf die Weichen entsprechend dem eingestellten Zielcode geschaltet werden. Die Zielcodes dienen sowohl zum Einschleusen eines Förderwagens von einer Nebenförderspur 6₁,6₂ in eine Hauptförderspur 8₁,8₂ wie umgekehrt. Im dargestellten Beispiel sind die Förderspuren Schienenstränge, es können aber auch am oder im Boden verlegte Leitspuren z.B. gemäss US-PSen 3 628 624, 3 948 342 und 4 015 680 sein, nach denen ein Förderwagen fährt. In der Hauptförderspur 8₁,8₂ sind Kehrweichen 10₁,10₂ angeordnet, um einen Förderwagen von der Hauptförderspur 8₁ in die Hauptförderspur 8₂ überzuführen bzw. umgekehrt.

Die Ueberwachungsanlage zum Nachrüsten einer solchen Förderanlage enthält nun Empfangsgeräte 12₁,12₂,12₃, welche einerseits an den Stationen A und B andererseits längs der Hauptförderspur 8₁,8₂ angeordnet sind. Solche Empfangsgeräte 12₁, 12₂,12₃ sind mit Leseeinrichtungen 14 für die Zielcodes sowie mit Leseeinrichtungen 16 für Identifikationscodes ausgerüstet, wobei jedem Förderwagen ein solcher individueller, den Förderwagen kennzeichnender Identifikationscode zugeordnet ist. Ein solcher Identifikationscode kann beispielsweise aus einem Barcode bestehen, der von einer optischen Leseeinrichtung 16 abgetastet wird. Der abgetastete Zielcode und der abgetastete Identifikationscode werden in dem Empfangsgerät mittels eines Mikroprozessors verarbeitet und über eine Verbindungsleitung 18₁,18₂,18₃ einer Datenbusleitung 20 eingespeist, die zu einem Zentralrechner 22 führt, der beispielsweise ein Personel-Computer ist. Am Zentralrechner 22 sind ein Bildschirm 24 und ein Drucker 26 angeschlossen.

Die Empfangsgeräte 12₁,12₂ an den Stationen A,B sind über eine Steuerleitung 28₁, 28₂ mit den jeweiligen Weichen 4₁,4₂ verbunden. Das längs der Hauptförderspur 8₁,8₂ angeordnete Empfangsgerät 12₃ kann über eine Steuerleitung 30 mit einer Codestellvorrichtung 32 und/oder über eine Steuerleitung 34 mit einer Kehrweiche 10₂ verbunden sein. Die Codestellvorrichtung 32 kann beispielsweise gemäss der US-PS 4 566 390 ausgestaltet sein und zum Zurückstellen der Codes in Ausgangsstellung dienen, wobei dann dieser zurückgestellte Code dem Zielcode einer Sammelstation entsprechen kann. Eine solche Codestellvorrichtung 32 kann aber auch gemäss der US-PS 4 056 063 oder der GB-PS 2 084 757 ausgebildet sein, die den Zielcode entweder auf einen solchen einer Sammelstation oder gegebenenfalls auf einen solchen einer Ausgangsstation einstellt.

Insbesondere die Empfangsgeräte 12₁,12₂ der Stationen A,B können mit akustischen oder optischen Alarmeinrichtungen 36 ausgestattet sein, die eine Fehlfunktion anzeigen, beispielsweise wenn die bestimmte Zielstation besetzt ist oder der eingestellte Zielcode nicht existiert.

Die Ueberwachungsanlage kann bei einer existierenden Förderanlage nachgerüstet werden, ohne dass die Steuertechnik der bestehenden Förderanlage selbst geändert werden muss. Die einzelnen Aggregate der Ueberwachungsanlage können einer bekannten Förderanlage zugefügt werden, wobei unter Umständen nicht einmal eine Betriebsunterbrechung notwendig wird. Die Ueberwachungsanlage übernimmt keine der Steueraufgaben der bekannten Förderanlage und wird unabhängig von dieser betrieben, das heisst also ergänzt lediglich die bekannte Förderanlage. Beim Ausfallen der Ueberwachungsanlage bleibt die Förderanlage funktionsfähig.

Die Zielcodes und die Identifikationscodes können nicht nur aus Schiebemagneten und Barcoden bestehen, sondern alle bekannten Codiereinrichtungen enthalten. Die Leseeinrichtungen können entsprechend ausgestaltet sein. Gegebenenfalls können die bestehenden Abtasteinrichtungen 2₁,2₂ für die Zielcodes direkt für die Leseeinrichtung 14 der Ueberwachungsanlage verwendet werden.

Mittels der Ueberwachungsanlage können die verschiedensten beispielsweise auch in der EP-OS 0 164 302 angesprochenen Aufgaben erfüllt werden:
1. Ermittlung der Fahrtzeitdauer jedes Förderauftrages und Vergleich dieses Wertes mit einem eingegebenen maximalen Zeitwert für die Erfüllung des Auftrages;
2. Feststellung ob ein Förderwagen das angegebene Ziel effektiv erreicht hat und Anzeige einer etwaigen falsch angefahrenen Station;
3. Fährt ein Förderwagen mit einem nicht in der Anlage vorgesehenen Zielcode, dann kann der Förderwagen an die Ausgangsstation zurückgeschickt oder an eine Sammelstation geleitet werden;
4. Fährt ein Förderwagen mehrmals an einem Empfangsgerät vorbei, so kann sein Zielcode durch die Codestellvorrichtung auf den Zielcode einer Sammelstation oder Ersatzstation geändert werden. Im Zentralrechner kann der ursprüngliche Zielcode gespeichert bleiben und an der Ersatzstation kann diese Information auf einem Drucker ausgegeben werden;
5. Sämtliche Bewegungsabläufe in der Förderanlage können in dem Zentralrechner für eine bestimmte Zeitdauer gespeichert und am Bildschirm angezeigt und am Drucker ausgedruckt werden;
6. Die im Zentralrechner gespeicherten Daten können nach verschiedenen Kriterien ausgesucht und ausgewertet werden, z.B. alle Bewegungen eines bestimmten Förderwagens in einer vorgegebenen Zeitspanne können angezeigt und ausgedruckt werden;
7. Die im Zentralrechner gespeicherten Daten können für Wartungsanweisungen ausgewertet werden;
8. Mit einer sinnvollen Verteilung von Empfangsgeräten längs der Förderanlage kann festgehalten und berechnet werden, welche Strecke ein Förderwagen bis zum Ziel durchfahren hat, ausserdem kann dadurch stets angezeigt werden, wo sich ein Behälter in der Förderanlage befindet. Fährt ein Förderwagen an einem längs des Streckenverlaufes angeordneten Empfangsgerät vorbei, so weiss man, dass bei jedem wiederholten Vorbeifahren ein berechenbarer Zusatzweg zu der Strecke addiert werden muss, die der Förderwagen bereits zurückgelegt hat. Fährt der Förderwagen mehrfach an einem durch ein Empfangsgerät gegebenen Kontrollpunkt vorbei und ist der Zielcode korrekt, dann bedeutet dies, dass die Zielstation besetzt war und der Förderwagen eine Zusatzrunde fahren muss.
9. Der Belegungszustand jeder Station kann festgestellt, angezeigt und berücksichtigt werden.

### Bezugszeichenliste

- A: Station
- B: Station
- 1: Förderwagen
- 2₁: Abtasteinrichtung
- 2₂: Abtasteinrichtung
- 4₁: Weiche
- 4₂: Weiche
- 6₁: Nebenförderspur
- 6₂: Nebenförderspur
- 8₁: Hauptförderspur
- 8₂: Hauptförderspur
- 10₁: Kehrweiche
- 10₂: Kehrweiche
- 12₁: Empfangsgeräte
- 12₂: Empfangsgeräte
- 12₃: Empfangsgeräte
- 14: Leseeinrichtung für Zielcode
- 16: Leseeinrichtung für Identifikationscode
- 18₁: Verbindungsleitung
- 18₂: Verbindungsleitung
- 18₃: Verbindungsleitung
- 20: Datenbusleitung
- 22: Zentralrechner
- 24: Bildschirm
- 26: Drucker
- 28₁: Steuerleitung
- 28₂: Steuerleitung
- 30: Steuerleitung
- 32: Codestellvorrichtung
- 34: Steuerleitung
- 36: Alarmeinrichtung

## Patentansprüche

1. Förderanlage mit spurgebundenen Förderwagen (1), die mittels an den Förderwagen einstellbarer Zielcodes von einer Station (A,B) entlang von Förderspuren (6₁,6₂,8₁,8₂) über Weichen (4₁,4₂,10₁,10₂) zu einer anderen Station (A,B) fahrbar sind, wobei den Weichen zu deren direkter Steuerung jeweils Abtasteinrichtungen (2₁,2₂) mit Leseeinrichtungen für die Zielcodes zugeordnet sind, dadurch gekennzeichnet, daß sie mit einer Ueberwachungsanlage zum Ueberwachen der Förderanlage versehen ist, welche:
- Empfangsgeräte (12₁,12₂), welche mit je einer Leseeinrichtung (16) zum Lesen von Identifikationscodes ausgerüstet sind, wobei jedem Förderwagen (1) ein solcher Identifikationscode zugeordnet ist;
- einen Zentralrechner (22), welcher mit den Empfangsgeräten (12₁,12₂) verbunden ist; und
- eine mit dem Zentralrechner (22) verbundene Codestellvorrichtung (32) umfaßt, welche längs der Förderspur eingebaut ist und zum Umprogrammieren des Zielcodes von längs der Förderspur (8₁,8₂) fahrenden Förderwagen aufgrund eines Steuersignals des Zentralrechners (22) dient.

2. Förderanlage nach Anspruch 1, dadurch gekennzeichnet, dass sie weitere mit dem Zentralrechner (22) verbundene längs der Förderspur (8₁,8₂) angeordnete Empfangsgeräte (12₃) jeweils mit einer Leseeinrichtung (16) für die Identifikationscodes sowie vorzugsweise mit einer Leseeinrichtung (14) für die Zielcodes aufweist.

3. Förderanlage nach Anspruch 1 oder 2, dadurch gekennzeichnet, dass die Empfangsgeräte (12₁,12₂,12₃) Mikroprozessoren enthalten.

4. Förderanlage nach einem der Ansprüche 1 bis 3, dadurch gekennzeichnet, dass die Empfangsgeräte (12₁,12₂, 12₃) zur Steuerung von Einrichtungen (4₁,4₂,32) in Abhängigkeit von Steuersignalen des Zentralrechners ausgebildet sind.

5. Förderanlage nach Anspruch 4, dadurch gekennzeichnet, dass die Empfangsgeräte (12₁,12₂) mit Steuereinrichtungen von Stationen und/oder Weichen (4₁,4₂,10₁,10₂) verbunden sind.

6. Förderanlage nach einem der Ansprüche 1 bis 5, dadurch gekennzeichnet, dass die Codestellvorrichtung (32) mit einem längs der Förderspur (8₁,8₂) angeordneten Empfangsgerät (12₃) verbunden ist.

7. Förderanlage nach einem der Ansprüche 1 bis 6, dadurch gekennzeichnet, dass am Zentralrechner (22) ein Bildschirm (24) und/oder Drucker (26) angeschlossen sind/ist.

## Claims

1. A conveying system with track-bound conveying cars (1) which may be moved from one station (A,B) along tracks (6₁,6₂,8₁,8₂), via switches (4₁,4₂,10₁,10₂), to another station (A,B) by means of adjustable target codes provided on the conveying cars, scanning devices (2₁,2₂) with reading devices for the target codes being associated, in each case, with the switches for the direct control of the cars, characterised in that the conveying system is provided with a monitoring system for monitoring the conveying system, comprising
- receivers (12₁,12₂) which are each provided with a reading device (16) for reading the identification codes, such an identification code being associated with each conveying car (1)
- a central computer (22) which is connected with the receivers (12₁,12₂); and
- a code-setting device (32) which is mounted along the track and is used for reprogramming the target codes of the cars running along the track (8₁,8₂), as a result of a control signal of the central computer (22).

2. A conveying system according to Claim 1, characterised in that it comprises other receivers (12₃) which are connected with the central computer (22) and are arranged along the track (8₁,8₂), and a reading device (16) for the identification codes and preferably a reading device (14) for the target codes.

3. A conveying system according to Claim 1 or 2, characterised in that the receivers (12₁,12₂,12₃) have microprocessors.

4. A conveying system according to any one of Claims 1 to 3, characterised in that the receivers (12₁,12₂,12₃) are constructed to control devices (4₁,4₂,32) as a function of control signals emitted by the central computer.

5. A conveying system according to Claim 4, characterised in that the receivers (12₁,12₂) are connected with control devices of stations and/or switches(4₁,4₂,10₁,10₂).

6. A conveying system according to any one of Claims 1 to 5, characterised in that the code-setting device (32) is connected with a receiver (12₃) arranged along the conveying track (8₁,8₂).

7. A conveying system according to any one of Claims 1 to 6, characterised in that a screen (24) and/or printer (26) is/are connected to the central computer (22).

## Revendications

1. Installation de transport, comportant des chariots guidés (1), qui, à l'aide d'un code cible adaptable aux chariots, peut se déplacer d'une station (A, B) à une autre station (A, B) le long de pistes (6₁, 6₂, 8₁, 8₂) en passant par des aiguillages (4₁, 4₂, 10₁, 10₂), un dispositif palpeur (2₁, 2₂) comportant des dispositifs de lecture du code cible étant attribué à chacun des aiguillages, pour permettre leur commande directe, caractérisée en ce qu'elle est pourvue d'une installation de surveillance destinée à surveiller l'installation de transport, et qui comporte :
- des appareils récepteurs (12₁, 12₂), dont chacun est pourvu d'un dispositif de lecture (16) destiné à lire le code d'identification, un code d'identification de ce type étant attribué à chaque chariot (1),
- un ordinateur central (22), qui est relié aux appareils récepteurs (12₁, 12₂) ; et
- un dispositif de codage (32), relié à l'ordinateur central (22), qui est installé le long de la piste et sert à reprogrammer le code cible de chariots se déplaçant le long de la piste (8₁, 8₂), en raison d'un signal de commande de l'ordinateur central (22).

2. Installation de transport selon la revendication 1, caractérisée en ce qu'elle comporte d'autres appareils récepteurs (12₃), reliés à l'ordinateur central (22) et disposés le long de la piste (8₁, 8₂), chacun d'eux comportant un dispositif de lecture (16) destiné à la lecture du code d'identification, et aussi, de préférence, un dispositif de lecture (14) servant à la lecture du code cible.

3. Installation de transport selon la revendication 1 ou 2, caractérisée en ce que les appareils récepteurs (12₁, 12₂, 12₃) contiennent des microprocesseurs.

4. Installation de transport selon l'une des revendications 1 à 3, caractérisée en ce que les appareils récepteurs (12₁, 12₂, 12₃) sont conçus pour commander des dispositifs (4₁, 4₂, 32) en fonction de signaux de commande de l'ordinateur central.

5. Installation de transport selon la revendication 4, caractérisée en ce que les appareils récepteurs (12₁, 12₂) sont reliés à des dispositifs de commande de stations et/ou d'aiguillages (4₁, 4₂, 10₁, 10₂).

6. Installation de transport selon l'une des revendications 1 à 5, caractérisée en ce que le dispositif de codage (32) est relié à un dispositif récepteur (12₃) disposé le long de la piste (8₁, 8₂).

7. Installation de transport selon l'une des revendications 1 à 6, caractérisée en ce qu'un écran (24) et/ou une imprimante (26) sont connectés à l'ordinateur central (22).
